(19) Europäisches Patentamt
European Patent Office
Office européen des brevets

(11) EP 1 690 526 A1

(12) **DEMANDE DE BREVET EUROPEEN**

(43) Date de publication:
**16.08.2006 Bulletin 2006/33**

(51) Int Cl.:
***A61K 8/89*** *(2006.01)* ***A61K 8/90*** *(2006.01)*

(21) Numéro de dépôt: **06290132.7**

(22) Date de dépôt: **19.01.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **15.02.2005 FR 0550430**

(71) Demandeur: **L'ORÉAL**
**75008 Paris (FR)**

(72) Inventeur: **Farcet, Céline**
**75012 Paris (FR)**

(74) Mandataire: **Dodin, Catherine**
**L'OREAL - D.I.P.I.**
**25-29 Quai Aulagnier**
**92600 Asnières (FR)**

(54) **Composition cosmétique comprenant une dispersion de particules de polymères, dispersion de particules de polymères et procédé comsétique l'utilisant**

(57) La présente demande concerne une composition cosmétique comprenant, dans un milieu cosmétiquement acceptable, au moins une dispersion de particules de polymères dans un milieu siliconé liquide, ledit polymère étant un copolymère comprenant au moins une première séquence soluble et au moins une deuxième séquence insoluble dans ledit milieu siliconé.

L'invention concerne également une telle dispersion de particules de polymères dans un milieu siliconé liquide, ainsi qu'un procédé cosmétique de maquillage, de nettoyage, de protection solaire, de mise en forme, de coloration, ou de soin des matières kératiniques, notamment de la peau du corps ou du visage, des ongles, des cheveux et/ou des cils, employant cette composition.

EP 1 690 526 A1

## Description

La présente invention a trait à de nouvelles dispersions de particules de polymères, et à leur utilisation en cosmétique; l'invention concerne aussi les compositions notamment cosmétiques comprenant ces dispersions.

Il est connu d'utiliser en cosmétique des dispersions de particules de polymère, généralement de taille nanométrique, dans des milieux organiques.
Ainsi, dans la demande de brevet européen EP-A-0749747, il est décrit une composition cosmétique comprenant une dispersion de particules de polymère, dans un milieu non aqueux, ladite dispersion étant stabilisée par ajout de polymères stabilisants, qui se lient de manière non covalente par le biais d'interactions physiques sur les particules de polymère.
Ce type de composition présente toutefois les inconvénients suivants : elle nécessite l'ajout dans le milieu non aqueux d'une quantité de polymères stabilisants supérieure à celle effectivement liée aux particules de polymères non solubles, afin d'obtenir une dispersion desdites particules relativement stable. Or, lors de l'ajout d'adjuvants tels que des pigments, dans les compositions, une partie des polymères stabilisants a tendance à se désorber des particules de polymères non solubles pour s'associer avec lesdits adjuvants, ce qui contribue à déstabiliser la dispersion, notamment par formation d'agglomérats entre les particules de polymères.
On connaît également, par la demande EP1428843, des compositions cosmétiques comprenant des dispersions, dans un milieu siliconé, de particules de polymères acryliques comprenant un squelette insoluble dans ledit milieu, et une partie soluble dans ledit milieu constituée de chaînes latérales liées de manière covalente audit squelette. Dans ce cas, les particules de polymères sont stabilisées par un polymère (macromère) qui est chimiquement lié aux particules de polymères.

Dans les deux cas, la nature du polymère stabilisant n'est pas très modulable, que cela soit en terme de nature chimique, de masse molaire et/ou d'architecture, et doit faire l'objet d'une synthèse spécifique. Par ailleurs, il n'est pas aisé de moduler les propriétés du coeur de la particule, que ce soit en terme de masse moléculaire et/ou d'architecture.

La demanderesse a découvert de manière surprenante de nouvelles dispersions de particules de polymères, permettant d'obtenir de bonnes propriétés cosmétiques telles qu'une bonne adhésion sur le support (peau ou cheveu, notamment) et donc une bonne tenue de la composition cosmétique.
Par ailleurs, la dispersion selon l'invention ne comprenant pas de stabilisant au sens de l'art antérieur, elle est beaucoup plus stable, dans le temps, que les dispersions usuelles, ce qui implique une meilleure stabilité de la composition la comprenant et une facilité de formulation.
Enfin, le confort de la composition est amélioré.

Un objet de la présente invention est une composition cosmétique comprenant, dans un milieu cosmétiquement acceptable, au moins une dispersion de particules de polymères dans un milieu siliconé liquide, ledit polymère étant un copolymère comprenant au moins une première séquence soluble dans ledit milieu siliconé et au moins une deuxième séquence insoluble dans ledit milieu siliconé.

Grâce à l'invention, il est possible de moduler les propriétés physico-chimiques de la dispersion, et donc de la composition la comprenant, en choisissant de manière adéquate les monomères et le milieu siliconé la composant, possibilité qui n'était pas envisageable avec l'art antérieur. Ceci permet notamment d'obtenir une dispersion qui présente une grande affinité pour les milieux huileux siliconés, usuellement employés en cosmétique; ceci permet également de préparer une dispersion dont le dépôt ne présente pas de collant.

Les micelles de copolymère-bloc, notamment en milieu organique carboné sont connues d'une manière générale. On peut notamment citer la demande de brevet WO01/77198 qui est relative à un procédé de préparation de microgels par polymérisation RAFT en présence d'un agent de transfert de chaîne, consistant à préparer un copolymère-bloc comprenant des monomères solvophobes et des monomères solvophiles, puis à disperser ledit copolymère-bloc dans un milieu de dispersion pour former des micelles, qui seront stabilisées pour donner le microgel attendu. Dans ce document, le milieu de dispersion peut être organique carboné, aqueux ou hydroorganique.
Toutefois, dans ce document, il n'est pas envisagé d'employer telles quelles les micelles ainsi préparées; elles servent à former des microgels par réticulation, lesdits microgels permettant d'encapsuler des pigments ou des colorants, par exemple, et étant susceptibles d'être employés dans divers domaines tels que le revêtement industriel.
Les microgels réticulés ainsi obtenus ne permettent pas d'obtenir des dépôts préférentiellement filmogènes, de bonne tenue et aisément démaquillables; or ceci constitue le principal objectif de la présente invention.
Par ailleurs, ce document ne mentionne nullement la possibilité de préparer une dispersion de particules dans les milieux siliconés.

Or, dans certains domaines de la cosmétique, le maquillage par exemple, les compositions comprennent généralement des corps gras siliconés, notamment des huiles de silicone et/ou des cires de silicone; or, l'introduction de particules de polymères, en dispersion dans des huiles hydrocarbonées, dans ces compositions est relativement peu aisé, et s'avère quasiment impossible lorsque la quantité d'huile siliconée est trop importante : on observe une perte de stabilité de la composition. Or, les huiles de silicone apportent de bonnes propriétés cosmétiques, telles qu'un toucher doux, un bon glissant de la composition et moins d'effet gras.

Il n'est donc pas souhaitable de s'affranchir de leur présence.
La présente invention permet de pallier ce problème et propose des dispersions de particules de polymère en milieu siliconé, qui permettent l'incorporation de grande quantité de particules de polymères dans des compositions cosmétiques à base de silicone, lesdites compositions présentant une bonne stabilité.

La dispersion de particules de polymères selon l'invention comprend donc un copolymère qui comprend au moins une première séquence soluble dans le milieu siliconé et au moins une deuxième séquence insoluble dans ledit milieu siliconé.

Par séquence, on entend dans la présente invention, un enchaînement polymérique, formé de plusieurs monomères, notamment d'au moins 5 monomères, identiques ou différents, et qui peut donc se présenter sous forme d'un homopolymère ou d'un copolymère statistique, alterné, à gradient ou bloc, notamment dibloc, tribloc ou multibloc.
De préférence, la séquence sera de type homopolymère ou à gradient.
Pour chaque séquence, le choix des monomères et de leur quantité, ainsi que de l'architecture de la séquence, pourra être effectué par l'homme du métier sur la base de ses connaissances générales de manière à obtenir au final une séquence ayant la solubilité requise (soluble ou insoluble) dans le milieu siliconé considéré.

Au final, le copolymère obtenu est de préférence du type 'dibloc', c'est-à-dire qu'il comprend uniquement deux séquences, l'une étant soluble dans le milieu, l'autre y étant insoluble; il peut toutefois être du type 'tribloc', voire 'multiblocs' (plus de trois blocs).
De préférence, l'enchaînement des blocs solubles et insolubles est alterné. Chaque bloc ou séquence soluble peut être de longueur et/ou de masse molaire différente ou identique, de nature chimique différente ou identique, d'architecture différente ou identique. Chaque bloc ou séquence insoluble peut être de longueur ou masse molaire différente ou identique, de nature chimique différente ou identique, d'architecture différente ou identique.

De préférence, le copolymère selon l'invention est linéaire; il peut toutefois être ramifié et/ou greffé.
De préférence, le copolymère selon l'invention n'est pas réticulé; il peut toutefois être réticulé, par ajout d'agent réticulant; dans ce cas, la séquence réticulée est de préférence la séquence insoluble, ce qui signifie que les particules finales peuvent avoir un coeur réticulé.

Par soluble, on entend que la séquence est complètement dissoute (sans dépôt apparent, ni agglomérat ou sédiment insoluble), visuellement, à 20°C, à une concentration supérieure ou égale à 5% en poids, dans le milieu siliconé considéré.

Les dispersions selon l'invention peuvent notamment se présenter sous forme de micelles polymériques (ou particules) en dispersion stable dans le milieu considéré. Ces micelles (ou particules) sont de préférence d'une taille comprise entre 5 et 1000 nm, de préférence de 10 à 500 nm, encore mieux de 20 à 300 nm, voire de 30 à 200 nm, ce qui permet d'obtenir une grande stabilité dans le temps de la dispersion.

Par micelles polymériques, on entend des particules autodispersées obtenues par auto-assemblage des copolymères tels que définis ci-après.
Ainsi, on peut considérer que la polymérisation du/des monomères composant la première séquence, d'amorceur et/ou d'agent de contrôle conduit à une première séquence soluble dans le milieu considéré. L'ajout du/des monomères destinés à composer le coeur de la particule aboutit à la formation du copolymère, généralement bloc, de type soluble/insoluble, copolymère qui va spontanément s'organiser en micelle polymérique, c'est-à-dire former une particule de polymère auto-dispersée en milieu siliconé.

Un des avantages lié à la présente invention est qu'il est ainsi possible de former en une seule étape des particules de copolymère dispersées, dont les caractéristiques de la partie soluble et celles du coeur de la particule sont simultanément contrôlables.

Les copolymères selon la présente invention ont de préférence un poids moléculaire moyen en nombre (Mn) compris entre 1000 à 700 000, notamment entre 10000 et 500 000, et encore mieux entre 15000 et 350 000, voire entre 30 000 et 150000.

De préférence, le copolymère selon l'invention présente un indice de polydispersité en masse (Ip) inférieur ou égal à 6 de préférence compris entre 1,05 et 4, notamment entre 1,1 et 3, voire entre 1,15 et 2,5.
L'indice de polydispersité en masse (Ip) du copolymère est égal au rapport de la masse moléculaire moyenne en poids (Mw) sur la masse moléculaire moyenne en nombre (Mn). Une faible polydispersité en masse traduit des longueurs de chaînes approximativement identiques.
On détermine les masses moléculaires moyennes en poids (Mw) et en nombre (Mn) par chromatographie liquide par perméation de gel (GPC), éluant THF, courbe d'étalonnage établie avec des étalons de polystyrène linéaire, détecteur réfractométrique.

De préférence, la dispersion selon l'invention présente une polydispersité en taille de particules homogène, ce qui signifie que toutes les particules sont de la même taille; notamment, la dispersion est de préférence telle qu'au moins 50% en nombre des particules de la dispersion ont un diamètre identique ou quasi-identique (différence inférieure à 10%); ceci contribue à une meilleure stabilité de la dispersion dans la temps (pas de décantation, floculation et/ou sédimentation).

Ceci est un avantage par rapport à certaines dispersions en milieu carboné de l'art antérieur, préparées selon les procédés de polymérisation radicalaire dits conventionnels; en effet, dans ces procédés, le produit obtenu est hétérogène en composition chimique car il s'agit généralement d'un mélange d'homopolymères et de copolymères.
Dans notre invention, la très grande majorité voire la totalité des chaînes (en fonction de la technique de polymérisation choisie) peut être sous forme de copolymère à blocs, ce qui aura pour particularité d'améliorer la stabilité des dispersions. Par ailleurs, les copolymères selon l'invention ont généralement des distributions en masse molaire et en compositions chimiques étroites et des masses molaires contrôlées ce qui permet de contrôler la taille des particules et leur distribution en taille.

Le copolymère selon l'invention comprend donc une première séquence soluble dans le milieu siliconé de dispersion et au moins une seconde séquence, insoluble dans ledit milieu.

La séquence soluble comprend de préférence 50 à 100% en poids de monomère(s) soluble(s) dans ledit milieu, notamment de 60 à 90% en poids, et encore mieux de 70 à 80% en poids de monomère(s) soluble(s), seul ou en mélange. Elle peut toutefois comprendre également 0 à 50% en poids, notamment de 10 à 40% en poids, voire de 20 à 30% en poids de monomère(s) insoluble(s) dans ledit milieu, seul ou en mélange.

De manière similaire, la séquence insoluble comprend de préférence 50 à 100% en poids de monomère(s) insoluble(s) dans ledit milieu, notamment de 60 à 90% en poids, et encore mieux de 70 à 80% en poids de monomère (s) insoluble(s), seul ou en mélange. Elle peut toutefois comprendre également 0 à 50% en poids, notamment de 10 à 40% en poids, voire de 20 à 30% en poids de monomère(s) soluble(s) dans ledit milieu, seul ou en mélange.

L'homme du métier saura choisir, sur la base de ses connaissances générales, le ou les monomères solubles et insolubles, ainsi que leurs quantités, pour obtenir au final une séquence ayant la solubilité requise (soluble ou insoluble) dans le milieu siliconé considéré.

Par monomère soluble dans le milieu, on entend tout monomère dont l'homopolymère est sous forme soluble c'est-à-dire complètement dissous à une concentration supérieure ou égale à 5% en poids à température ambiante (20°C) dans ledit milieu.
Par monomère insoluble, on entend donc tout monomère dont l'homopolymère n'est pas sous forme soluble c'est-à-dire complètement dissous à une concentration supérieure ou égale à 5% en poids à température ambiante (20°C) dans ledit milieu. Toutefois, les monomères insolubles peuvent, en tant que monomères, être solubles dans le milieu considéré, étant entendu qu'ils deviennent insolubles après polymérisation.

En tout état de cause, la proportion de séquence soluble et de séquence insoluble dans le copolymère doit être telle que le copolymère puisse former une micelle polymérique.
De préférence, la séquence insoluble (ou les séquences insolubles), représente 15 à 97% en poids du poids total du copolymère, notamment de 30 à 95% en poids, voire de 50 à 93% en poids, notamment 60 à 92% en poids, et encore mieux de 75 à 90% en poids.
La séquence soluble (ou les séquences solubles), représente donc de préférence 3 à 85% en poids du poids total du copolymère, notamment de 5 à 70% en poids, voire de 7 à 50% en poids,notamment 8 à 40% en poids, et encore mieux de 10 à 25% en poids.

Comme monomère soluble susceptible d'être employé, on peut citer, seul ou en mélange, les monomères suivants :

- les monomères éthyléniques dont le groupement ester contient des silanes, des silsesquioxanes, des siloxanes, des carbosiloxanes dendrimères tels que ceux décrits dans le brevet EP963751, à l'exception des monomères ne contenant qu'un atome de silicium tel que le méthacryloxypropryl trimethoxysilane. Des monomères préférés sont le (méth)acryloxypropyltris(trimethylsiloxy)silane, le (méth)acryloxypropylbis(trimethylsiloxy)méthylsilane, le (méth) acryloxyméthyltris(trimethylsiloxy)silane, et le (méth)acryloxyméthylbis(trimethylsiloxy)méthylsilane.

- les macromonomères PDMS, tels que les polydiméthylsiloxanes à groupement terminal monoacryloyloxy ou monométhacryloyloxy, et notamment ceux de formule suivante :

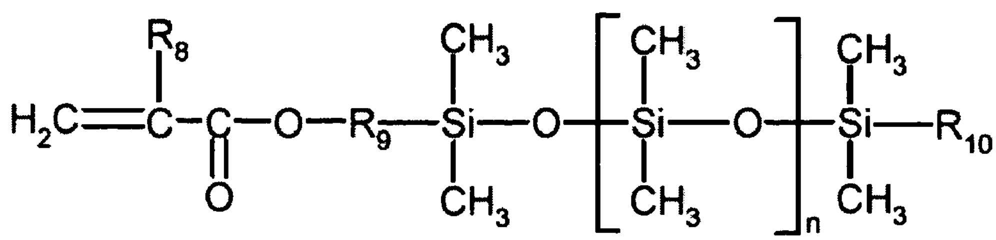

dans laquelle :

- R8 désigne un atome d'hydrogène ou un groupement méthyle; de préférence méthyle;
- R9 désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;
- R10 désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100. On peut en particulier utiliser les monométhacryloyloxypropyl polydiméthylsiloxanes tels que ceux commercialisés sous la dénomination PS560-K6 par UCT (United Chemical Technologies Inc.) ou sous la dénomination MCR-M17 par Gelest Inc.

Comme monomère soluble particulièrement préféré, on peut citer le (méth)acryloxypropyltris(trimethylsiloxy) silane, le (méth)acryloxypropylbis(trimethylsiloxy)méthylsilane, le (méth)acryloxyméthyltris(trimethylsiloxy)silane, et le (méth)acryloxyméthylbis(trimethylsiloxy)méthylsilane et les monométhacryloyloxypropyl polydiméthylsiloxanes; et leurs mélanges.

Comme monomère insoluble susceptible d'être employé, on peut citer, seul ou en mélange, les monomères suivants, ainsi que leurs sels et leurs mélanges:

- (i) les (méth)acrylates de formule : $CH_2=C(CH_3)-COOR_1$ ou $CH_2=CH-COOR_1$

dans laquelle R1 représente un groupe choisi parmi :

- un groupe alkyle, linéaire ou ramifié, comprenant de 1 à 30 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène (F, Cl, Br, I) et -NR'R'' avec R' et R'', identiques ou différents, choisis parmi les alkyles, linéaires ou ramifiés, en C1-C4; et/ou pouvant être substitué par au moins un groupe polyoxyalkylène, en particulier avec un alkylène en C2-C4, notamment un polyoxyéthylène et/ou un polyoxypropylène, ledit groupe polyoxyalkylène étant constitué par la répétition de 5 à 30 motifs oxyalkylène;
- un groupe alkyle cyclique comprenant de 3 à 30 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I);

A titre d'exemples de R1, on peut citer le groupe méthyle, éthyle, propyle, butyle, tertiobutyle, isobutyle, lauryle, stéaryle, béhényle, 2-éthylhexyle, méthoxyéthyle, éthoxyéthyle, méthoxy-polyoxyéthylène 30, trifluoroéthyle, 2-hydroxyéthyle, 2-hydroxypropyle, diméthylaminoéthyle, diéthylaminoéthyle, diméthylaminopropyle, isobornyle.

- (ii) les (méth)acrylamides de formule : $CH_2=C(CH_3)-CONR_3R_4$ ou $CH_2=CH-CONR_3R_4$,

dans laquelle :

- R3 et R4, identiques ou différents, représentent un atome d'hydrogène ou un groupe alkyle, linéaire ou ramifié, comportant de 1 à 12 atomes de carbone, pouvant comporter un ou plusieurs substituants choisis parmi -OH, les atomes d'halogène (F, Cl, Br, I) et -NR'R'' avec R' et R'', identiques ou différents, choisis parmi les alkyles, linéaires ou ramifiés, en C1-C4;ou

- R3 représente un atome d'hydrogène et R4 représente un groupe 1,1-diméthyl-3-oxobutyle ; A titre d'exemples de groupes alkyles pouvant constituer R3 et R4, on peut citer le n-butyle, le t-butyle, le n-propyle, l'isooctyle, l'isononyle, l'undecyle, le diméthylaminoéthyle, le diéthylaminoéthyle, le diméthylaminopropyle.

- (iii) les monomères à insaturation(s) éthylénique(s) comprenant au moins une fonction acide carboxylique, phosphorique ou sulfonique, tel que l'acide crotonique, l'anhydride maléique, l'acide itaconique, l'acide fumarique, l'acide maléique, l'acide styrènesulfonique, l'acide vinylbenzoïque, l'acide vinylphosphorique, l'acide acrylique, l'acide méthacrylique, l'acide acrylamidopropanesulfonique, et leurs sels;

- (iv) les esters de vinyle de formule : R6-COO-CH=CH2 dans laquelle R6 représente un groupe alkyle linéaire ou ramifié, comprenant de 1 à 22 atomes de carbone, ou un groupe alkyle cyclique comportant de 3 à 6 atomes de

carbone et/ou un groupe aromatique, par exemple de type benzénique, anthracénique, et naphtalénique ;

- (v) les monomères à insaturation(s) éthylénique(s) comprenant au moins une fonction amine tertiaire, tel que la 2-vinylpyridine, la 4-vinylpyridine, et leurs mélanges.

- (vi) le styrène et ses dérivés ;

- (vii) les oligopeptides fonctionnalisés par une fonction (méth)acrylate.

- (viii) les éthers d'alcool vinylique et d'alcool de formule $R_6O-CH=CH_2$ dans laquelle $R_6$ représente un groupe alkyle linéaire ou ramifié, comprenant de 1 à 22 atomes de carbone ;

- (ix) les monomères éthyléniques dont le groupement ester contient des silanes ou des siloxanes, et ne contenant qu'un atome de silicium tel que le (méth)acryloxypropyl trimethoxysilane;

- (x) des macromonomères carbonés ayant un groupe terminal polymérisable;
  On entend par "macromonomère ayant un groupe terminal polymérisable" tout oligomère comportant sur une seule de ses extrémités un groupe terminal polymérisable apte à réagir lors de la réaction de polymérisation avec des monomères éthyléniques. Le groupe polymérisable du macromonomère peut être avantageusement un groupe à insaturation éthylénique susceptible de se polymériser par voie radicalaire. Ledit groupe terminal polymérisable peut être en particulier un groupe vinyle ou (méth)acrylate (ou (méth)acryloxy), et de préférence un groupe (méth) acrylate. Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques.
  Comme macromonomères carbonés ayant un groupe terminal polymérisable, on peut en particulier citer :

  - (a) les homopolymères et les copolymères (méth)acrylate d'alkyle linéaire ou ramifié en C6-C22, de préférence en C8-C18, présentant un groupe terminal polymérisable choisi parmi les groupes vinyle ou (méth)acrylate parmi lesquels on peut citer en particulier: les macromonomères de poly(acrylate d'éthyl-2 hexyle) à extrémité mono(méth)acrylate; les macromonomères de poly(acrylate de dodécyle) ou de poly(méthacrylate de dodécyle) à extrémité mono(méth)acrylate ; les macromonomères de poly(acrylate de stéaryle) ou de poly (méthacrylate de stéaryle) à extrémité mono(méth)acrylate.
    De tels macromonomères sont notamment décrits dans les brevets EP895467 et EP96459 et dans l'article Gillman , Polymer Letters, Vol 5, page 477-481 (1967).
    On peut en particulier citer les macromonomères à base de poly(acrylate d'éthyl-2-hexyle) ou de poly(acrylate de dodécyle) à extrémité mono(méth)acrylate.
  - (b) les polyoléfines ayant un groupe terminal à insaturation éthylénique, en particulier ceux ayant un groupement terminal (méth)acrylate. Comme exemple de telles polyoléfines, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène ;
    De tels macromonomères sont en particulier décrits dans EP 1347013 ou encore dans US 5,625,005 qui mentionne des macromonomères éthylène/butylène et éthylène/propylène à groupement terminal réactif (méth) acrylate. On peut en particulier citer le méthacrylate de poly(éthylène/butylène), tel que celui commercialisé sous la dénomination Kraton Liquid L-1253 par Kraton Polymers.

- (xi) les di-n-alkylitaconates de formule $CH_2=C(CH_2-COO(CH_2)_{n-1}-CH_3)-COO(CH_2)_{n-1}-CH_3$, avec n étant un entier supérieur ou égal à 1, notamment compris entre 1 et 12.

Parmi les sels, on peut citer ceux obtenus par neutralisation des groupements acides à l'aide de base inorganiques telles que l'hydroxyde de sodium, l'hydroxyde de potassium, l'hydroxyde d'ammonium ou de bases organiques de type alcanols amines comme la monoéthanolamine, la diéthanolamine, la triéthanolamine, la 2-méthyl-2-amino-1-propanol.
On peut également citer les sels formés par neutralisation des motifs amine tertiaire, par exemple à l'aide d'acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides comportant un

ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique ou l'acide propionique, l'acide téréphtalique, ainsi que l'acide citrique et l'acide tartrique.

Comme monomère insoluble particulièrement préféré, on peut citer, seul ou en mélange :

- les (méth)acrylates et en particulier les (méth)acrylates de méthyle, d'éthyle, de propyle, de butyle, d'isopropyle, de tertiobutyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle; le (méth) acrylate d'éthyle-2-hexyle, le (méth)acrylate d'isobornyle, le (méth)acrylate de lauryle, le (méth)acrylate de stéaryle, le (méth)acrylate de béhényle, ainsi que leurs sels;
- les monomères à insaturation(s) éthylénique(s) comprenant au moins une fonction acide carboxylique, et notamment l'acide (méth)acrylique et ses sels; ou l'anhydride maléïque.

Plus particulièrement, on peut citer les (méth)acrylates de méthyle, d'éthyle, l'acide (méth)acrylique et l'anhydride maléïque.

La dispersion de particules de polymères selon l'invention comprend également un milieu siliconé liquide dans lequel sont dispersées lesdites particules.

Par milieu liquide, on entend notamment un milieu ayant de préférence une viscosité inférieure ou égale à 7000 centipoises à 20°C.

Selon l'invention, le milieu est dit siliconé, s'il comprend au moins 50% en poids, notamment de 50,1 à 100% en poids, par exemple de 60 à 99% en poids, ou encore de 65 à 95% en poids, voire de 70 à 90% en poids, par rapport au poids total du milieu siliconé, de composé siliconé, liquide à 25°C, ayant un paramètre de solubilité global selon l'espace de solubilité de HANSEN inférieur ou égal à 20 $(MPa)^{1/2}$, ou d'un mélange de tels composés.

Le paramètre de solubilité global $\delta$ selon l'espace de solubilité de HANSEN est défini dans l'article "Solubility parameter values" de Grulke, dans l'ouvrage "Polymer Handbook" 3ème édition, Chapitre VII, pages 519-559 par la relation :

$$\delta = ( d_D^2 + d_P^2 + d_H^2)^{1/2}$$

dans laquelle :

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.). La définition des solvants dans l'espace de solubilité tridimensionnel selon HANSEN est décrite dans l'article de HANSEN : "The three dimensionnal solubility parameters" J. Paint Technol. 39, 105 (1967).

Parmi les composés siliconés liquides ayant un paramètre de solubilité global selon l'espace de solubilité de HANSEN inférieur ou égal à 20 $(MPa)^{1/2}$, on peut citer les huiles siliconées, volatiles ou non volatiles, seules ou en mélange.

Par huile volatile, on entend une huile susceptible de s'évaporer de la peau ou des lèvres en moins d'une heure, ayant notamment une pression de vapeur, à température ambiante et pression atmosphérique allant de $10^{-3}$ à 300 mm de Hg (0,13 Pa à 40 000 Pa).

Parmi les huiles siliconées non volatiles, on peut notamment citer :

- les polydialkylsiloxanes non volatils, tels que les polydiméthylsiloxanes (PDMS) non volatils, éventuellement substitués par des groupements aliphatiques et/ou aromatiques, éventuellement fluorés, et/ou comportant des groupements fonctionnels tels que des groupements hydroxyles, thiols et/ou amines; et notamment les polysiloxanes modifiés par des acides gras (notamment en C8-C20), des alcools gras (notamment en C8-C20) ou des polyoxyalkylènes (notamment polyoxyéthyléne et/ou polyoxypropylène); les polysiloxanes aminées ; les polysiloxanes à groupement hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor
- les polydiméthylsiloxanes comportant des groupements alkyle, alcoxy ou phényle, pendant ou en bout de chaîne

siliconée, ces groupements pouvant avoir de 2 à 24 atomes de carbone;

- les silicones phénylées comme les phényl triméthicones, les phényl diméthicones, les phényl triméthylsiloxy diphénylsiloxanes, les diphényl diméthicones, les diphényl méthyldiphényl trisiloxanes; et également les polyméthylphénylsiloxanes, éventuellement substitués par des groupements aliphatiques et/ou aromatiques, éventuellement fluorés, et/ou comportant des groupements fonctionnels tels que des groupements hydroxyles, thiols et/ou amines;
- leurs mélanges.

Parmi les huiles siliconées volatiles, on peut citer les huiles siliconées cycliques ou linéaires, ayant 2 à 7 atomes de silicium, et comportant éventuellement des groupes alkyle ou alkoxy ayant de 1 à 10 atomes de carbone, telles que les cyclodiméthylsiloxanes, les cyclophénylmethylsiloxanes et les diméthylsiloxanes linéaires, et notamment la dodecamethylpentasiloxane linéaire (L5), l'octaméthylcyclotétrasiloxane (D4), le décaméthylcyclopentasiloxane (D5), le dodécaméthylcyclohexasiloxane (D6), l'heptaméthylhexyltrisiloxane, l'heptaméthyloctyltrisiloxane, l'octaméthyltrisiloxane, le décaméthyltétrasiloxane, et leurs mélanges.

De préférence, la dispersion comprend dans le milieu siliconé, au moins un composé siliconé choisi parmi, seul ou en mélange :

- les silicones phénylées et notamment les phényl triméthicones, les phényl diméthicones, et les polyméthylphénylsiloxanes;
- les huiles siliconées volatiles, cycliques ou linéaires, ayant 2 à 7 atomes de silicium, et notamment la dodecamethylpentasiloxane linéaire (L5), l'octaméthylcyclotétrasiloxane (D4), le décaméthylcyclopentasiloxane (D5), le dodécaméthylcyclohexasiloxane (D6), et leurs mélanges.

Une dispersion comprenant des particules de polymères dans un milieu siliconé est nouvelle et constitue, en tant que telle, un objet de la présente invention.

Le milieu siliconé peut éventuellement comprendre des composés liquides additionnels qui peuvent être présents en une quantité strictement inférieure à 50% en poids, notamment de 1 à 40% en poids, voire de 5 à 35% en poids, ou encore de 10 à 30% en poids, par rapport au poids total du milieu siliconé, et choisis parmi, seul ou en mélange :

- les huiles végétales formées par des esters d'acides gras et de polyols, en particulier les triglycérides, telles que l'huile de tournesol, de sésame ou de colza, de macadamia, de soja, l'huile d'amande douce, de calophyllum, de palme, de pépins de raisin, de maïs, d'arara, de coton, d'abricot, d'avocat, de jojoba, d'olive ou de germes de céréales;
- les esters linéaires, ramifiés ou cycliques, ayant 2 à 30 atomes de carbone; et notamment l'isononanoate d'isononyle ; et plus particulièrement les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle ;
- les hydrocarbures et notamment les alcanes linéaires, ramifiés et/ou cycliques, volatils ou non volatils, tels que les isoparaffines en $C_5$-$C_{60}$ , éventuellement volatiles tels que l'isododécane, le Parléam (polyisobutène hydrogéné), l'isohexadécane, le cyclohexane, ou les 'ISOPARs'; ou bien les huiles de paraffine, de vaseline, ou le polyisobutylène hydrogéné.
- les éthers ayant 2 à 30 atomes de carbone ;
- les cétones ayant 2 à 30 atomes de carbone;
- les monoalcools, notamment gras, aliphatiques ayant 1 à 30 atomes de carbone, la chaîne hydrocarbonée ne comportant pas de groupement de substitution, tels que l'alcool oléique, le décanol, le dodécanol, l'octadécanol, l'octyldodécanol et l'alcool linoléique;
- les polyols notamment ayant 6 à 30 atomes de carbone, tels que l'hexylène glycol;
- leurs mélanges.

Préférentiellement, les composés liquides additionnels, lorsqu'ils sont présents, sont choisis parmi, seul ou en mélange :

- les esters linéaires, ramifiés ou cycliques, ayant 2 à 30 atomes de carbone; et notamment l'isononanoate d'isononyle, l'adipate de diisopropyle et le myristate d'isopropyle ;
- les alcanes linéaires, ramifiés et/ou cycliques, volatils ou non volatils, tels que les isoparaffines en $C_5$-$C_{60}$, éventuellement volatiles tels que l'isododécane, le Parléam (polyisobutène hydrogéné), l'isohexadécane, le cyclohexane, ou les 'ISOPARs'; ou bien les huiles de paraffine, de vaseline, ou le polyisobutylène hydrogéné;
- les monoalcools gras aliphatiques ayant 8 à 30 atomes de carbone, la chaîne hydrocarbonée ne comportant pas de groupement de substitution, tels que l'alcool oléique, le décanol, le dodécanol, l'octadécanol, l'octyldodécanol

et l'alcool linoléique.

Toutefois, selon un mode particulier de réalisation de l'invention, le milieu siliconé ne contient pas de composés liquides additionnels.
Le choix du milieu siliconé peut être effectué aisément par l'homme du métier en fonction de la nature des monomères constituant le polymère et/ou de la destination de la composition.
Parmi les dispersions de diblocs tout particulièrement préférées, on peut citer les dispersions de particules :

- de poly(méthacryloxypropyltris(trimethylsiloxy)silane)-b-poly(acrylate de méthyle),
- de poly(méthacryloxypropyltris(trimethylsiloxy)silane)-b-poly(acrylate de méthyle-co-acide acrylique),
- de poly(méthacryloxypropyltris(trimethylsiloxy)silane-co-acide acrylique)-b-poly(acrylate de méthyle),
- de poly(acryloxypropyltris(trimethylsiloxy)silane)-b-poly(acrylate de méthyle),
- de poly(acryloxypropyltris(trimethylsiloxy)silane)-b-poly(acrylate de méthyle-co-acide acrylique),
- de poly(acryloxypropyltris(trimethylsiloxy)silane-co-acide acrylique)-b-poly(acrylate de méthyle),
- de poly(méthacryloxypropylbis(trimethylsiloxy)méthylsilane)-b-poly(acrylate de méthyle),
- de poly(méthacryloxypropylbis(trimethylsiloxy)méthylsilane)-b-poly(acrylate de méthyle-co-acide acrylique),
- de poly(méthacryloxypropylbis(trimethylsiloxy)méthylsilane-co-acide acrylique)-b-poly(acrylate de méthyle),
- de poly(acryloxypropylbis(trimethylsiloxy)méthylsilane)-b-poly(acrylate de méthyle),
- de poly(acryloxypropylbis(trimethylsiloxy)méthylsilane)-b-poly(acrylate de méthyle-co-acide acrylique),
- de poly(acryloxypropylbis(trimethylsiloxy)méthylsilane-co-acide acrylique)-b-poly(acrylate de méthyle),
- de poly(polydimethylsiloxane méthacryloyloxy)-b-poly(acrylate de méthyle),
- de poly(polydimethylsiloxane méthacryloyloxy)-b-poly(acrylate de méthyle-co-acide acrylique),
- de poly(polydimethylsiloxane méthacryloyloxy-co-acide acrylique)-b-poly(acrylate de méthyle),
- de poly(polydimethylsiloxane méthacryloyloxy-co-méthacryloxypropyltris-(trimethylsiloxy)silane)-b-poly(acrylate de méthyle),
- de poly(polydimethylsiloxane méthacryloyloxy-co-méthacryloxypropyltris-(trimethylsiloxy)silane)-b-poly(acrylate de méthyle-co-acide acrylique),
- de poly(polydimethylsiloxane méthacryloyloxy-co-méthacryloxypropyltris-(trimethylsiloxy)silane-co-acide acrylique)-b-poly(acrylate de méthyle),
- de poly(polydimethylsiloxane méthacryloyloxy-co-acryloxypropyltris-(trimethylsiloxy)silane)-b-poly(acrylate de méthyle),
- de poly(polydimethylsiloxane méthacryloyloxy-co-acryloxypropyltris-(trimethylsiloxy)silane)-b-poly(acrylate de méthyle-co-acide acrylique),
- de poly(polydimethylsiloxane méthacryloyloxy-co-acryloxypropyltris-(trimethylsiloxy)silane-co-acide acrylique)-b-poly(acrylate de méthyle),
- de poly(méthacryloxypropylbis(trimethylsiloxy)méthylsilane-co-méthacryloxypropyltris(trimethylsiloxy)silane)-b-poly(acrylate de méthyle),
- de poly(méthacryloxypropylbis(trimethylsiloxy)méthylsilane-co-méthacryloxypropyltris(trimethylsiloxy)silane)-b-poly(acrylate de méthyle-co-acide acrylique),
- de poly(méthacryloxypropylbis(trimethylsiloxy)méthylsilane-co-méthacryloxypropyltris(trimethylsiloxy)silane-co-acide acrylique)-b-poly(acrylate de méthyle),
- de poly(méthacryloxypropylbis(trimethylsiloxy)méthylsilane-co-acryloxypropyltris(trimethylsiloxy)silane)-b-poly (acrylate de méthyle),
- de poly(méthacryloxypropylbis(trimethylsiloxy)méthylsilane-co-acryloxypropyltris(trimethylsiloxy)silane)-b-poly (acrylate de méthyle-co-acide acrylique),
- de poly(méthacryloxypropylbis(trimethylsiloxy)méthylsilane-co-acryloxypropyltris(trimethylsiloxy)silane-co-acide acrylique)-b-poly(acrylate de méthyle),
- de poly(méthacryloxy-propyltris(trimethylsiloxy)silane-co-acryloxypropyltris(trimethylsiloxy)silane)-b-poly(acrylate de méthyle),
- de poly(méthacryloxy-propyltris(trimethylsiloxy)silane-co-acryloxypropyltris(trimethylsiloxy)silane)-b-poly(acrylate de méthyle-co-acide acrylique),
- de poly(méthacryloxy-propyltris(trimethylsiloxy)silane-co-acryloxypropyltris(trimethylsiloxy)silane-co-acide acrylique)-b-poly(acrylate de méthyle),
- de poly(polydimethylsiloxane méthacryloyloxy-co-méthacryloxypropylbis(trimethylsiloxy)méthylsilane)-b-poly (acrylate de méthyle)
- de poly(polydimethylsiloxane méthacryloyloxy-co-méthacryloxypropylbis(trimethylsiloxy)méthylsilane)-b-poly (acrylate de méthyle-co-acide acrylique)
- de poly(polydimethylsiloxane méthacryloyloxy-co-méthacryloxypropylbis(trimethylsiloxy)méthylsilane-co-acide

acrylique)-b-poly(acrylate de méthyle);

- de poly(polydimethylsiloxane méthacryloyloxy-co-acryloxypropylbis(trimethylsiloxy)méthylsilane)-b-poly(acrylate de méthyle)
- de poly(polydimethylsiloxane méthacryloyloxy-co-acryloxypropylbis(trimethylsiloxy)méthylsilane)-b-poly(acrylate de méthyle-co-acide acrylique)
- de poly(polydimethylsiloxane méthacryloyloxy-co-acryloxypropylbis(trimethylsiloxy)méthylsilane-co-acide acrylique)-b-poly(acrylate de méthyle);

dans un milieu siliconé tel que défini ci-dessus, plus particulièrement dans une silicone phénylée et/ou une huile de silicone volatile, et notamment dans une huile de silicone volatile cyclique telle que la D5.

Parmi les dispersions de triblocs tout particulièrement préférées, on peut citer les dispersions de particules :

- de poly(méthacryloxypropyltris(trimethylsiloxy)silane)-b-poly(acrylate de méthyle)-b- poly(méthacryloxypropyltris (trimethylsiloxy)silane,
- de poly(méthacryloxypropyltris(trimethylsiloxy)silane-co-acide acrylique)-b-poly(acrylate de méthyle)-b- poly(méthacryloxypropyltris(trimethylsiloxy)silane-co-acide acrylique),
- de poly(méthacryloxypropyltris(trimethylsiloxy)silane)-b-poly(acrylate de méthyle-co-acide acrylique)-b- poly(méthacryloxypropyltris(trimethylsiloxy)silane),
- de poly(acryloxypropyltris(trimethylsiloxy)silane)-b-poly(acrylate de méthyle)-b-poly(acryloxypropyltris(trimethylsiloxy)silane),
- de poly(acryloxypropyltris(trimethylsiloxy)silane-co-acide acrylique)-b-poly(acrylate de méthyle)-b- poly(acryloxypropyltris(trimethylsiloxy)silane-co-acide acrylique),
- de poly(acryloxypropyltris(trimethylsiloxy)silane)-b-poly(acrylate de méthyle-co-acide acrylique)-b- poly(acryloxypropyltris(trimethylsiloxy)silane),
- de poly(méthacryloxypropylbis(trimethylsiloxy)méthylsilane)-b-poly(acrylate de méthyle)-b- poly(méthacryloxypropylbis(trimethylsiloxy)méthylsilane);
- de poly(méthacryloxypropylbis(trimethylsiloxy)méthylsilane-co-acide acrylique)-b-poly(acrylate de méthyle)-b- poly (méthacryloxypropylbis(trimethylsiloxy)-méthylsilane-co-acide acrylique);
- de poly(méthacryloxypropylbis(trimethylsiloxy)méthylsilane)-b-poly(acrylate de méthyle-co-acide acrylique)-b- poly (méthacryloxypropylbis(trimethylsiloxy) méthylsilane)
- de poly(acryloxypropylbis(trimethylsiloxy)méthylsilane)-b-poly(acrylate de méthyle)-b- poly(acryloxypropylbis(trimethylsiloxy)méthylsilane)
- de poly(acryloxypropylbis(trimethylsiloxy)méthylsilane-co-acide acrylique)-b-poly(acrylate de méthyle)-b- poly (acryloxypropylbis(trimethylsiloxy)méthylsilane-co-acide acrylique)
- de poly(acryloxypropylbis(trimethylsiloxy)méthylsilane)-b-poly(acrylate de méthyle-co-acide acrylique)-b- poly (acryloxypropylbis(trimethylsiloxy)méthylsilane)
- de poly(polydimethylsiloxane méthacryloyloxy)-b-poly(acrylate de méthyle)-b-poly(polydimethylsiloxane méthacryloyloxy)
- de poly(polydimethylsiloxane méthacryloyloxy-co-acide acrylique)-b-poly(acrylate de méthyle)-b- poly(polydimethylsiloxane méthacryloyloxy-co-acide acrylique)
- de poly(polydimethylsiloxane méthacryloyloxy)-b-poly(acrylate de méthyle-co-acide acrylique)-b- poly(polydimethylsiloxane méthacryloyloxy)
- de poly(polydimethylsiloxane méthacryloyloxy-co-méthacryloxypropyltris-(trimethylsiloxy)silane)-b-poly(acrylate de méthyle)-b-poly(polydimethylsiloxane méthacryloyloxy-co-méthacryloxypropyltris-(trimethylsiloxy)silane)
- de poly(polydimethylsiloxane méthacryloyloxy-co-méthacryloxypropyltris-(trimethylsiloxy)silane-co-acide acrylique)-b-poly(acrylate de méthyle)-b-poly(polydimethylsiloxane méthacryloyloxy-co-méthacryloxypropyltris-(trimethylsiloxy)silane-co-acide acrylique)
- de poly(polydimethylsiloxane méthacryloyloxy-co-méthacryloxypropyltris-(trimethylsiloxy)silane)-b-poly(acrylate de méthyle-co-acide acrylique)-b-poly(polydimethylsiloxane méthacryloyloxy-co-méthacryloxypropyltris-(trimethylsiloxy)silane)
- de poly(polydimethylsiloxane méthacryloyloxy-co-acryloxypropyltris-(trimethylsiloxy)silane)-b-poly(acrylate de méthyle)-b-poly(polydimethylsiloxane méthacryloyloxy-co-acryloxypropyltris-(trimethylsiloxy)silane)
- de poly(polydimethylsiloxane méthacryloyloxy-co-acryloxypropyltris-(trimethylsiloxy)silane-co-acide acrylique)-b-poly(acrylate de méthyle)-b-poly(polydimethylsiloxane méthacryloyloxy-co-acryloxypropyltris-(trimethylsiloxy)silane-co-acide acrylique),
- de poly(polydimethylsiloxane méthacryloyloxy-co-acryloxypropyltris-(trimethylsiloxy)silane)-b-poly(acrylate de méthyle-co-acide acrylique)-b- poly(poly-dimethylsiloxane méthacryloyloxy-co-acryloxypropyltris-(trimethylsiloxy)silane),

- de poly(méthacryloxy-propyltris(trimethylsiloxy)silane-co-acryloxypropyltris-(trimethylsiloxy)silane)-b-poly(acrylate de méthyle)-b-poly(méthacryloxypropyltris(trimethylsiloxy)silane-co-acryloxy-propyltris(trimethylsiloxy)silane)
- de poly(méthacryloxy-propyltris(trimethylsiloxy)silane-co-acryloxypropyltris-(trimethylsiloxy)silane-co-acide acrylique)-b-poly(acrylate de méthyle)-b-poly(méthacryloxy-propyltris(trimethylsiloxy)silane-co-acryloxypropyltris(trimethylsiloxy)silane-co-acide acrylique)
- de poly(méthacryloxypropyltris(trimethylsiloxy)silane-co-acryloxypropyltris(trimethylsiloxy)silane)-b-poly(acrylate de méthyle-co-acide acrylique)-b-poly(méthacryloxypropyltris(trimethylsiloxy)silane-co-acryloxypropyltris(trimethylsiloxy)silane).

dans un milieu siliconé tel que défini ci-dessus, plus particulièrement dans une silicone phénylée et/ou une huile de silicone volatile, et notamment dans une huile de silicone volatile cyclique telle que la D5.

La dispersion selon l'invention présente de préférence un taux de matière sèche compris entre 5 et 80% en poids, notamment 8 à 70% en poids, voire 10 à 60%, ou encore 15 à 50% en poids, et mieux 18 à 25% en poids.

La dispersion de polymère peut être fabriquée par tout moyen connu de l'homme du métier, et notamment par polymérisation radicalaire contrôlée ou par polymérisation vivante, notamment par les techniques dites nitroxydes/alcoxyamines, ATRP, aux organoCobalt, RAFT/MADIX, transfert dégénératif, TERP (tellurium), au Selenium, par Iniferter, ou par tout procédé de polymérisation vivante (anionique ou cationique), par métallocène, par ROMP (ring opening metathesis polymerization), par ROP (ring opening polymerization) cationique ou anionique, par GTP (group transfer polymerization), par les dérivés du tétraphenyléthane, par le diphénylethylène. Les techniques employées pour la formation de chaque séquence peuvent être identiques ou différentes.

Un procédé type peut consister à préparer la première séquence, dite soluble, dans le milieu siliconé de la dispersion, par polymérisation du ou des monomères, d'un agent de contrôle et d'un amorceur si nécessaire. Ensuite, le ou les monomères de la séquence dite insoluble sont ajoutés en présence ou non d'amorceur. La température de réaction est de préférence comprise entre -30 et 200°C, de préférence de 0 à 160°C et plus préférentiellement de 40 à 140°C. Des séquences supplémentaires peuvent être polymérisées selon le même procédé. Pour chacune des séquences, le ou les monomères peuvent être ajoutés simultanément, en batch, en semi-continu ou consécutivement. On obtiendra alors des polymères multiblocs.

Si la première séquence, dite soluble, est synthétisée en masse, la séquence dite insoluble peut être ensuite synthétisée en masse ou en solution. Le solvant peut être un solvant siliconé tel que défini dans la présente demande, ce qui conduit à l'issue de la synthèse du copolymère à une dispersion directement dans le milieu siliconé. Le solvant employé peut également être un solvant commun à toutes les séquences; dans ce cas, l'ajout ultérieur d'un solvant siliconé tel que défini ci-dessus dans la présente demande et l'élimination éventuelle du solvant commun conduira à la dispersion voulue dans le milieu siliconé.

Si tout le copolymère est synthétisé en masse, l'ajout d'un solvant siliconé tel que défini ci-dessus conduira à la dispersion voulue.

Si toutes les séquences sont synthétisées en solution, dans un solvant commun, l'ajout ultérieur d'un solvant siliconé tel que défini ci-dessus et l'élimination éventuelle du solvant commun conduira à la dispersion voulue dans les milieux siliconés. Il est également possible à ce stade d'éliminer le solvant commun afin de récupérer le polymère seul et avant de le disperser dans un solvant siliconé tel que défini ci-dessus, ce qui conduira à la dispersion voulue.

Enfin, si toutes les séquences sont synthétisées directement dans un solvant siliconé tel que défini ci-dessus, la dispersion est obtenue directement, en une seule étape. Ce dernier procédé est celui utilisé de façon préférentielle.

Une fois la dispersion obtenue, il est possible de changer de milieu siliconé par élimination, puis ajout d'un nouveau solvant siliconé, ou bien par ajout puis élimination éventuelle du premier solvant.

De préférence, la première séquence est préparée par polymérisation radicalaire contrôlée (PRC), la seconde séquence pouvant être également préparée par PRC ou par polymérisation conventionnelle.

Un second mode opératoire préféré consiste à synthétiser la séquence soluble en masse, puis à la solubiliser dans un solvant siliconé selon l'invention, puis à synthétiser la séquence insoluble dans ce solvant siliconé; on obtient ainsi directement une dispersion du polymère dans le solvant siliconé.

Dans un mode de réalisation particulier de l'invention, une fois la dispersion obtenue, il est possible d'y ajouter un ou plusieurs monomères C dont les homopolymères sont soit de type solubles, soit de type insolubles, selon la définition donnée plus haut, et de préférence insolubles, dans le milieu, afin de continuer la polymérisation sur les copolymères à blocs A-B déjà formés, ce qui conduit à la formation de copolymères triblocs A-B-C.

Le ou les monomères additionnels C peuvent être présents en une quantité telle que les quantités de monomères solubles et insolubles totales restent dans les fourchettes totales mentionnées ci-dessus.

Dans le cas où le copolymère de départ est un tribloc de structure A-B-A, la polymérisation de C peut conduire à un pentabloc de structure C-A-B-A-C ou A-B-C-B-A, selon la technique de polymérisation et/ou l'agent de transfert employé.

L'amorceur de polymérisation peut être tout amorceur connu de l'homme de l'art pour la polymérisation radicalaire (peroxydes, azoïques, couple rédox, photochimique). Dans le cas de certaines techniques de polymérisation

radicalaire contrôlée, un même composé peut avoir pour rôle d'amorcer la polymérisation et d'être l'agent de contrôle comme c'est le cas des alcoxyamines. Pour les polymérisations non radicalaires, c'est-à-dire ioniques (anionique ou cationique), l'homme de l'art peut choisir l'amorceur adéquat.

Pendant la polymérisation, il est possible de réticuler les particules de la dispersion soit au coeur (bloc insoluble), soit dans l'écorce (bloc soluble), soit dans les deux.

De préférence, la réticulation est faite au coeur de la particule de manière à obtenir un dépôt filmogène une fois le solvant de la dispersion évaporé. Parmi les comonomères susceptibles d'être employés pour la réticulation pendant la polymérisation et/ou après la polymérisation par activation, on peut citer le méth)acrylate d'allyle, le (méth)acrylate de cinnamoyle, le (méth)acrylate de glycidyle, l'anhydride maléïque, les monomères contenant une fonction latérale isocyanate qui après ajout de diamine ou de dialcool va former respectivement une liaison urée ou uréthane.

La réticulation peut également être obtenue par post-réticulation de la fonction silane Si-H ou Si-alkyl par hydrolyse en milieu protique; un monomère préféré est dans ce cas le méthacryloxypropyltriméthoxysilane.

La réticulation pendant la polymérisation est de préférence réalisée par ajout de comonomères difonctionnels, de préférence de type diacrylate ou diméthacrylate ou méthacrylate d'allyle.

On obtient ainsi des copolymères qui s'auto-organisent en dispersion dans le milieu considéré. Ils sont composés d'une première séquence soluble A et d'au moins une seconde séquence, insoluble, B, ce qui va provoquer une auto-organisation des chaînes de polymères de manière à former des particules présentant à l'interface avec le milieu les séquences A et au coeur de la particule les séquences B. Une fois, la dispersion obtenue, il est possible d'y ajouter des dispersants, des stabilisants, pour en modifier les propriétés physicochimiques (viscosité, Tg, etc).

Les dispersions selon l'invention trouvent une application toute particulière en cosmétique. Ainsi, elles peuvent être présentes dans les compositions cosmétiques selon l'invention en une quantité de 0,1 à 90% en poids, de préférence 0,5 à 80% en poids, notamment 1 à 75% en poids, voire 5-70% en poids, de dispersion par rapport au poids total de la composition.

Les compositions cosmétiques selon l'invention comprennent en outre, un milieu cosmétiquement acceptable, c'est-à-dire compatible avec les matières kératiniques telles que la peau du visage ou du corps, les lèvres, les cheveux, les cils, les sourcils et les ongles.

La composition peut avantageusement comprendre une phase grasse, qui peut elle-même comprendre des huiles et/ou des solvants de préférence lipophiles, ainsi que des corps gras solides à température ambiante tels que les cires, les corps gras pâteux, les gommes et leurs mélanges.

Parmi les constituants de la phase grasse, on peut citer les huiles, volatiles ou non, qui peuvent être choisies parmi les huiles naturelles ou synthétiques, carbonées, hydrocarbonées, fluorées, éventuellement ramifiées, seules ou en mélange.

On entend par "huile non volatile", une huile susceptible de rester sur la peau à température ambiante et pression atmosphérique au moins une heure et ayant notamment une pression de vapeur à température ambiante (25°C) et pression atmosphérique, non nulle, inférieure à 0,01 mm de Hg (1,33 Pa).

On peut en particulier citer les huiles non volatile carbonées, notamment hydrocarbonées, d'origine végétale, minérale, animale ou synthétique, telles que l'huile de paraffine (ou vaseline), le squalane, le polyisobutène hydrogéné (Parléam), le perhydrosqualène, l'huile de vison, de macadamia, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales, de beurre de karité; les esters linéaires, ramifiés ou cycliques, ayant plus de 6 atomes de carbone, notamment 6 à 30 atomes de carbone, tels que les esters d'acide lanolique, d'acide oléique, d'acide laurique, d'acide stéarique; les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, en particulier les esters en C12-C36, tels que le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine; les acides gras supérieurs, notamment en C14-C22, tels que l'acide myristique, l'acide palmitique, l'acide stéarique, l'acide béhénique, l'acide oléique, l'acide linoléique, l'acide linolénique ou l'acide isostéarique; les alcools gras supérieurs, notamment en C16-C22, tels que le cétanol, l'alcool oléique, l'alcool linoléique ou linolénique, l'alcool isostéarique ou l'octyl dodécanol; et leurs mélanges.

On peut encore citer le décanol, le dodécanol, l'octadécanol, les triglycérides liquides d'acides gras de 4 à 10 atomes de carbone comme les triglycérides des acides heptanoïque ou octanoïque, les triglycérides des acides caprylique/caprique; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés

comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol.

On peut encore citer les cétones liquides à température ambiante tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyléther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à température ambiante tels que le diéthyléther, le diméthyléther ou le dichlorodiéthyléther; les alcanes liquides à température ambiante tels que le décane, l'heptane, le dodécane, l'isododécane, l'isohexadécane, le cyclohexane; les composés cycliques aromatiques liquides à température ambiante tels que le toluène et le xylène; les aldéhydes liquides à température ambiante tels que le benzaldéhyde, l'acétaldéhyde et leurs mélanges.

Parmi les composés volatils, on peut citer les huiles volatiles non siliconées, notamment les isoparaffines en C8-C16 comme l'isododécane, l'isodécane, l'isohexadécane. Plus préférentiellement, on peut citer les alcanes liquides à température ambiante, volatils ou non, et plus particulièrement le décane, l'heptane, le dodécane, l'isododécane, l'isohexadécane, le cyclohexane, l'isodécane, et leurs mélanges.

La phase grasse peut être présente en une teneur allant de 0,01 à 95%, de préférence de 0,1 à 90%, de préférence encore de 10 à 85% en poids, par rapport au poids total de la composition, et mieux de 30 à 80%.

La composition peut également comprendre, une phase hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le pentylène glycol, et les polyéthylène glycols, ou bien encore des éthers en $C_2$ et des aldéhydes en $C_2$-$C_4$ hydrophiles. L'eau ou le mélange d'eau et de solvants organiques hydrophiles peut être présent dans la composition selon l'invention en une teneur allant de 0,1 à 80% en poids, par rapport au poids total de la composition, et de préférence de 1 à 70% en poids.

La composition selon l'invention peut également comprendre des cires et/ou des gommes. Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25°C et mieux supérieure à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0,01 à 50% en poids de cires, par rapport au poids total de la composition et mieux de 1 à 30 % en poids.

La composition selon l'invention peut en outre comprendre une ou des matières colorantes choisies parmi les colorants hydrosolubles, les colorants liposolubles, et les matières colorantes pulvérulentes comme les pigments, les nacres, et les paillettes bien connues de l'homme du métier. Les matières colorantes peuvent être présentes, dans la composition, en une teneur allant de 0,01 à 50% en poids, par rapport au poids de la composition, de préférence de 0,01 à 30% en poids.

Par pigments, il faut comprendre des particules de toute forme, blanches ou colorées, minérales ou organiques, insolubles dans le milieu physiologique, destinées à colorer la composition. Par nacres, il faut comprendre des particules de toute forme irisées, notamment produites par certains mollusques dans leur coquille ou bien synthétisées. Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les

poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium. Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.
Parmi les colorants hydrosolubles, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition. Les charges peuvent être minérales ou organiques de toute forme, plaquettaires, sphériques ou oblongues, quelle que soit la forme cristallographique (par exemple feuillet, cubique, hexagonale, orthorhombique, etc.). On peut citer le talc, le mica, la silice, le kaolin, les poudres de polyamide (Nylon®) (Orgasol® de chez Atochem), de poly-p-alanine et de polyéthylène, les poudres de polymères de tétra-fluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition selon l'invention peut également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents antipelliculaires, les agents propulseurs, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter notamment sous forme de suspension, de dispersion, de solution notamment organique, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan). La composition peut être anhydre, par exemple il peut s'agir d'une pâte anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).
La composition selon l'invention peut être une composition de protection ou de soin de la peau du visage, du cou, des mains ou du corps, notamment une composition anti-rides, anti-fatigue permettant de donner un coup d'éclat à la peau, une composition hydratante ou traitante; une composition anti-solaire ou de bronzage artificiel.
La composition selon l'invention peut être également un produit capillaire, notamment pour le maintien de la coiffure ou

la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsque l'on souhaite obtenir un spray, une mousse pour la fixation ou le traitement des cheveux.

L'invention a aussi pour objet un procédé cosmétique de maquillage, de nettoyage, de protection solaire, de mise en forme, de coloration, ou de soin des matières kératiniques, notamment de la peau du corps ou du visage, des ongles, des cheveux et/ou des cils, comprenant l'application sur lesdites matières d'une composition cosmétique telle que définie précédemment.

L'invention est illustrée plus en détails dans les exemples suivants, donnés à titre d'illustration.

## Exemples 1 et 2

### **1/ Synthèse** du bloc soluble

Liste des abréviations :

**AMe :** Acrylate de Méthyle
**ASi4 :** 3-acryloxypropyltris(trimethylsiloxy)silane
**D5 :** cyclopentadimethylsiloxane
**MeSi4 :** methacryloxypropyltris(trimethylsiloxy)silane
**PAMe** : Poly(acrylate de méthyle)
**PASi4** : Poly(3-acryloxypropyltris(trimethylsiloxy)silane)
**PMeSi4** : Poly(methacryloxypropyltris(trimethylsiloxy)silane)

Les différents composants du mélange (monomère, agent de transfert, amorceur) sont mis en présence et homogénéisés par agitation dans un ballon de contenance 10 ml muni d'un septum. Le mélange est dégazé par bullage d'azote durant 5 minutes, puis le volume mort du ballon est soumis à un fort courant d'azote durant quelques secondes. Le ballon est ensuite introduit dans un bain d'huile thermostaté à 80°C. Il en est retiré et refroidi sous courant d'eau froide après 7h. Les blocs ainsi obtenus sont précipités deux fois à froid dans le méthanol, puis séchés sous cloche à vide.

| **Bloc 1a** | Monomère | Agent de transfert | Amorceur | Conversion | Mn théorique* | Mn exp. (g/mol) /Ip |
|---|---|---|---|---|---|---|
| nature | 3-acryloxy-propyltris-(trimethylsiloxy) silane | DTB | T21S | | | 12000 / 1.08 |
| masse | 2 g | 21,3 mg | 8 mg | 72% | 14400 | |
| **Bloc 1b** | Monomère | Comonomère | Agent de transfert | Amorceur | Mn théorique* | Mn exp. (g/mol) /Ip |
| nature | methacryloxy-propyl- tris (trimethylsiloxy) silane | Styrène | DTB | T21 S | | 33000 / 1.25 |
| masse | 1.51 g | 38 mg | 15.8 mg | 6.1 mg | 20100 | |
| DTB: Dithiobenzoate de tertiobutyle<br>T21 S : Trigonox 21 S, tert-butyl peroxy-2-ethylhexanoate<br>* : Mn théorique en g/mol, à conversion totale du bloc soluble | | | | | | |

**2/ Synthèse** du bloc insoluble, formation de dispersions

Les différents composants du mélange (monomère, bloc soluble, amorceur, solvant) sont mis en présence et homogénéisés par agitation dans un ballon rotaflo® muni d'un septum. Le mélange est dégazé par bullage d'azote durant 5 minutes, puis le volume mort du ballon est soumis à un fort courant d'azote durant quelques secondes. Le ballon est ensuite introduit dans un bain d'huile thermostaté à 80°C. Il en est retiré et refroidi sous courant d'eau froide après des temps variables de réaction. Une dispersion est ainsi obtenue.

| Exem ple 1 | Bloc soluble | Amorceur | Monomère | Solvant | Durée/taux de conversion | Mn théorique** | Mn exp.*** (g/mol) / Ip. |
|---|---|---|---|---|---|---|---|
| nature | Bloc 1a | T21S | acrylate de méthyle | D5 | | | 3000 / 1.23 |
| masse | 252 mg | 101.8 mg* | 914 mg | 3.35 g | 5 h / 14% | 6200 | |
| *: solution de T21 S à 6.5 $10^{-2}$ mol/L dans l'AMe<br>** : Mn théorique du bloc insoluble en g/mol, à la conversion obtenue expérimentalement<br>*** : Mn expérimentale du bloc insoluble en g/mol, Ip du copolymère à bloc | | | | | | | |

| Exemple 2 | Bloc soluble | Amorceur | Monomère | Solvant | Durée | Mn théorique** | Mn exp.*** (g/mol) / Ip. |
|---|---|---|---|---|---|---|---|
| nature | Bloc 1b | T21 S | acrylate de méthyle | D5 | | | 7000 / 1.25 |
| masse | 233 mg | 102.8 mg* | 191 mg | 1.06 g | 18 h | 42000 | |
| * : solution de T21 S à 2 ,6 $10^{-2}$ mol/L dans l'acrylate de méthyle<br>** : Mn théorique du bloc insoluble en g/mol, à conversion totale<br>*** : Mn expérimentale du bloc insoluble en g/mol, Ip du copolymère à bloc | | | | | | | |

**Exemple 3 : caractérisations des dispersions**

| Exemple | Extrait sec théorique | diamètre des particules | % massique de fraction soluble | % molaire de fraction soluble |
|---|---|---|---|---|
| 1 | 30% | 18 nm | 80% | 45.7% |
| 2 | 42% | 20 nm | 82.5% | 50% |

Les conversions sont mesurées par RMN $^{1}H$.
Les masses molaires par GPC dans le THF avec des étalons de polystyrènes linéaires.
Les diamètres moyens des particules sont mesurés par diffusion dynamique de la lumière avec un Malvern Nano-S90 en tenant compte de l'indice de réfraction et de la viscosité du solvant.

**Exemple 4 : Composition de mascara**

On a préparé un mascara ayant la composition suivante :

| | |
|---|---|
| Cires | 17 g |
| Hectorite modifiée (Bentone® 38V d'Elementis) | 5,3 g |
| Carbonate de propylène | 1,7 g |
| Charge | 1 g |
| pigments | 5 g |

(suite)

| | |
|---|---|
| Dispersion de polymère de l'exemple 1 | 12 g MS* |
| Isododécane | qsp 100 g |

*MS : matière sèche

Le mascara, après application sur les cils, est jugé très satisfaisant.

**Exemple 5 : Stick de rouge à lèvres**

La composition de rouge à lèvres suivante est préparée :

| | |
|---|---|
| Cire | 15 % |
| Dispersion de polymère de l'exemple 2 | 10 % en MS |
| Huile carbonée non volatile | 26 % |
| Pigments | 8.6 % |
| Isododécane | qsp 100% |

La composition obtenue après application sur les lèvres présente de bonnes propriétés cosmétiques.

**Exemple 6 : Fond de teint EIH**

On prépare une composition de fond de teint comprenant les composés suivants :

| | |
|---|---|
| Phase A | |
| Cetyl Dimethicone copolyol (ABIL EM 90 de la société GOLDSCHMIDT) | 3 g |
| Succinate d'isostéaryl diglycéryle (IMWITOR 780K de la société CONDEA) | 0,6 g |
| Isododécane | 18,5 g |
| Pigments (oxydes de fer et oxydes de titane hydrophobes) | 10 g |
| Dispersion de polymère de l'exemple 1 | 8 g en MS |
| Charge | 8 g |
| Parfum | qs |
| Phase B | |
| Eau | qsp 100 g |
| Sulfate de magnésium | 0,7 g |
| Conservateur (Methylparaben) | qs |
| Phase C | |
| Eau | 2 g |
| Conservateur (Diazolinyl urée) | qs |

La composition obtenue présente de bonnes propriétés cosmétiques.

**Exemple 7: Poudre compactée**

On prépare une poudre compactée ayant la composition suivante :

*Composition A :*

| | |
|---|---|
| - Talc | 30 g |
| - Oxychlorure de bismuth | 10 g |
| - Stéarate de zinc | 4 g |
| - Poudre de Nylon | 20 g |
| - Dispersion de l'exemple 2 | 5 g |

(suite)

| | |
|---|---|
| *Composition B :* | |
| - Oxydes de fer | 2 g |
| - Huile de vaseline | 6 g |

La poudre est obtenue de la façon suivante : on broie la composition A dans un broyeur de type KENWOOD pendant environ 5 minutes sous faible agitation, on ajoute la composition B et on broie l'ensemble environ 2 minutes à la même vitesse, puis 3 minutes à une vitesse plus rapide. On tamise ensuite la préparation sur un tamis de 0,16 mm, puis on compacte ce mélange dans des coupelles.
On obtient une poudre compactée présentant de bonnes propriétés cosmétiques.
La composition obtenue est aisée et agréable à appliquer. On constate que le film ne migre pas dans les ridules de la peau, même après avoir été porté pendant plusieurs heures.

__Exemple 8 : gel pour le visage__

On prépare la composition suivante :

| | |
|---|---|
| . isopropyl palmitate | 10 g |
| . vaseline (cire) | 5 g |
| . hectorite modifiée (argile) | 0,15 g |
| . ozokérite (cire) | 5 g |
| . septaoléate de sorbitane oxyéthyléné (400E) | 5 g |
| . dispersion de l'exemple 1 (25% de MS) | 75 g |

On obtient un gel ayant de bonnes propriétés cosmétiques.

__Exemple 9 : huile de soin__

On prépare la composition suivante :

| | |
|---|---|
| . dispersion de l'exemple 2(25% de MS) | 70 g |
| . huile de jojoba | 15 g |
| . huile de soja | 15 g |

On obtient une huile de soin qui peut être appliquée sur le corps ou le visage.

## Revendications

1. Composition cosmétique comprenant, dans un milieu cosmétiquement acceptable, au moins une dispersion de particules de polymères dans un milieu siliconé liquide, ledit polymère étant un copolymère comprenant au moins une première séquence soluble dans ledit milieu siliconé et au moins une deuxième séquence insoluble dans ledit milieu siliconé.

2. Composition selon la revendication 1, dans laquelle le copolymère présente un indice de polydispersité en masse (Ip) inférieur ou égal à 6 de préférence compris entre 1,05 et 4, notamment entre 1,1 et 3, voire entre 1,15 et 2,5.

3. Composition selon l'une des revendications précédentes, dans laquelle chaque séquence est de type homopolymère ou à gradient.

4. Composition selon l'une des revendications précédentes, dans laquelle le copolymère est du type 'dibloc', 'tribloc' ou 'multiblocs'.

5. Composition selon l'une des revendications précédentes, dans laquelle le copolymère est linéaire et non réticulé.

**6.** Composition selon l'une des revendications précédentes, dans laquelle les particules sont d'une taille comprise entre 5 et 1000 nm, de préférence 10 à 500 nm, encore mieux 20 à 300 nm, voire 30 à 200 nm.

**7.** Composition selon l'une des revendications précédentes, dans laquelle le copolymère a un poids moléculaire moyen en nombre (Mn) compris entre 1000 à 700 000, notamment entre 10000 et 500 000, et encore mieux entre 15000 et 350 000, voire entre 30 000 et 150 000.

**8.** Composition selon l'une des revendications précédentes, dans laquelle la séquence soluble comprend 50 à 100% en poids de monomère(s) soluble(s) dans ledit milieu, notamment de 60 à 90% en poids, et encore mieux de 70 à 80% en poids de monomère(s) soluble(s), seul ou en mélange.

**9.** Composition selon l'une des revendications précédentes, dans laquelle la séquence insoluble comprend 50 à 100% en poids de monomère(s) insoluble(s) dans ledit milieu, notamment de 60 à 90% en poids, et encore mieux de 70 à 80% en poids de monomère(s) insoluble(s), seul ou en mélange.

**10.** Composition selon l'une des revendications précédentes, dans laquelle la séquence insoluble (ou les séquences insolubles), représente 15 à 97% en poids du poids total du copolymère, notamment de 30 à 95% en poids, voire de 50 à 93% en poids, et encore mieux 60 à 92% en poids, et encore mieux de 75 à 90% en poids; et la séquence soluble (ou les séquences solubles) représente 3 à 85% en poids du poids total du copolymère, notamment de 5 à 70% en poids, voire de 7 à 50% en poids,notamment 8 à 40% en poids, et encore mieux de 10 à 25% en poids.

**11.** Composition selon l'une des revendications précédentes, dans laquelle le copolymère comprend au moins un monomère soluble choisi parmi, seul ou en mélange, les monomères suivants :

- les monomères éthyléniques dont le groupement ester contient des silanes, des silsesquioxanes, des siloxanes, des carbosiloxanes dendrimères, à l'exception des monomères ne contenant qu'un atome de silicium tel que le méthacryloxypropryl trimethoxysilane;
- les macromonomères PDMS, tels que les polydiméthylsiloxanes à groupement terminal monoacryloyloxy ou monométhacryloyloxy, et notamment ceux de formule suivante :

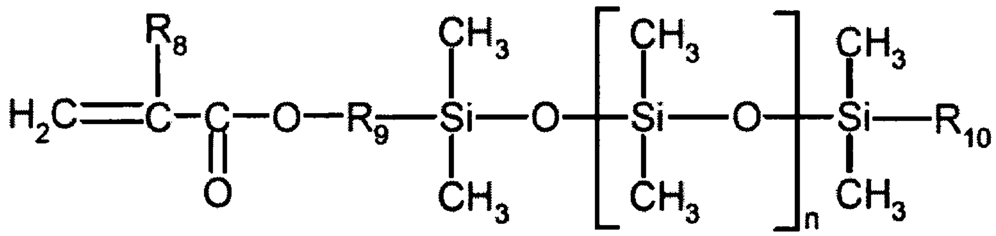

dans laquelle :

- R8 désigne un atome d'hydrogène ou un groupement méthyle; de préférence méthyle;
- R9 désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;
- R10 désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.

**12.** Composition selon la revendication 11, dans laquelle le monomère soluble est choisi parmi le (méth)acryloxypropyltris (trimethylsiloxy)sitane, le (méth)acryloxy-propylbis(trimethylsiloxy)méthylsilane, le (méth)acryloxyméthyltris-(trimethylsiloxy)silane, et le (méth)acryloxyméthylbis(trimethylsiloxy)méthylsilane et les monométhacryloyloxypropyl polydiméthylsiloxanes; et leurs mélanges.

**13.** Composition selon l'une des revendications précédentes, dans laquelle le copolymère comprend au moins un monomère insoluble choisi parmi, seul ou en mélange, les monomères suivants, ainsi que leurs sels et leurs mélanges:

- (i) les (méth)acrylates de formule : $CH_2=C(CH_3)-COOR$, ou $CH_2=CH-COOR$, dans laquelle R1 représente un groupe choisi parmi :

    - un groupe alkyle, linéaire ou ramifié, comprenant de 1 à 30 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène (F, Cl, Br, I) et -NR'R" avec R' et R", identiques ou différents, choisis parmi les alkyles, linéaires ou ramifiés, en C1-C4; et/ou pouvant être substitué par au moins un groupe polyoxyalkylène, en particulier avec un alkylène en C2-C4, notamment un polyoxyéthylène et/ou un polyoxypropylène, ledit groupe polyoxyalkylène étant constitué par la répétition de 5 à 30 motifs oxyalkylène;
    - un groupe alkyle cyclique comprenant de 3 à 30 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I);

- (ii) les (méth)acrylamides de formule : $CH_2=C(CH_3)-CONR_3R_4$ ou $CH_2=CH-CONR_3R_4$, dans laquelle :

    - R3 et R4, identiques ou différents, représentent un atome d'hydrogène ou un groupe alkyle, linéaire ou ramifié, comportant de 1 à 12 atomes de carbone, pouvant comporter un ou plusieurs substituants choisis parmi -OH, les atomes d'halogène (F, Cl, Br, I) et -NR'R" avec R' et R", identiques ou différents, choisis parmi les alkyles, linéaires ou ramifiés, en C1-C4;ou
    - R3 représente un atome d'hydrogène et R4 représente un groupe 1,1-diméthyl-3-oxobutyle ;

- (iii) les monomères à insaturation(s) éthylénique(s) comprenant au moins une fonction acide carboxylique, phosphorique ou sulfonique, tel que l'acide crotonique, l'anhydride maléique, l'acide itaconique, l'acide fumarique, l'acide maléique, l'acide styrènesulfonique, l'acide vinylbenzoïque, l'acide vinylphosphorique, l'acide acrylique, l'acide méthacrylique, l'acide acrylamidopropanesulfonique, et leurs sels;
- (iv) les esters de vinyle de formule : R6-COO-CH=CH2 dans laquelle R6 représente un groupe alkyle linéaire ou ramifié, comprenant de 1 à 22 atomes de carbone, ou un groupe alkyle cyclique comportant de 3 à 6 atomes de carbone et/ou un groupe aromatique, par exemple de type benzénique, anthracénique, et naphtalénique ;
- (v) les monomères à insaturation(s) éthylénique(s) comprenant au moins une fonction amine tertiaire, tel que la 2-vinylpyridine, la 4-vinylpyridine, et leurs mélanges.
- (vi) le styrène et ses dérivés ;
- (vii) les oligopeptides fonctionnalisés par une fonction (méth)acrylate.
- (viii) les éthers d'alcool vinylique et d'alcool de formule $R_6O-CH=CH_2$ dans laquelle $R_6$ représente un groupe alkyle linéaire ou ramifié, comprenant de 1 à 22 atomes de carbone ;
- (ix) les monomères éthyléniques dont le groupement ester contient des silanes ou des siloxanes, et ne contenant qu'un atome de silicium tel que le (méth)acryloxypropyl trimethoxysilane;
- (x) des macromonomères carbonés ayant un groupe terminal polymérisable tels que :

    - (a) les homopolymères et les copolymères (méth)acrylate d'alkyle linéaire ou ramifié en C6-C22, de préférence en C8-C18, présentant un groupe terminal polymérisable choisi parmi les groupes vinyle ou (méth)acrylate parmi lesquels on peut citer en particulier: les macromonomères de poly(acrylate d'éthyl-2 hexyle) à extrémité mono(méth)acrylate; les macromonomères de poly(acrylate de dodécyle) ou de poly (méthacrylate de dodécyle) à extrémité mono(méth)acrylate ; les macromonomères de poly(acrylate de stéaryle) ou de poly (méthacrylate de stéaryle) à extrémité mono(méth)acrylate.
    - (b) les polyoléfines ayant un groupe terminal à insaturation éthylénique, en particulier ceux ayant un groupement terminal (méth)acrylate, et notamment les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène ;

- (xi) les di-n-alkylitaconates de formule $CH_2=C(CH_2-COO(CH_2)_{n-1}-CH_3)-COO(CH_2)_{n-1}-CH_3$, avec n étant un entier supérieur ou égal à 1, notamment compris entre 1 et 12.

**14.** Composition selon la revendication 13, dans laquelle le monomère insoluble est choisi parmi, seul ou en mélange :

- les (méth)acrylates et en particulier les (méth)acrylates de méthyle, d'éthyle, de propyle, de butyle, d'isopropyle, de tertiobutyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle; le (méth)acrylate d'éthyle-2-hexyle, le (méth)acrylate d'isobornyle, le (méth)acrylate de lauryle, le (méth) acrylate de stéaryle, de béhényle, ainsi que leurs sels;
- les monomères à insaturation(s) éthylénique(s) comprenant au moins une fonction acide carboxylique, et notamment l'acide (méth)acrylique et ses sels, ou l'anhydride maléïque.

**15.** Composition selon la revendication 14, dans laquelle le monomère insoluble est choisi parmi le (méth)acrylate de méthyle, le (méth)acrylate d'éthyle, l'anhydride maléïque et l'acide (méth)acrylique.

**16.** Composition selon l'une des revendications précédentes, dans laquelle le milieu siliconé comprend au moins 50% en poids, notamment de 50,1 à 100% en poids, par exemple de 60 à 99% en poids, ou encore de 65 à 95% en poids, voire de 70 à 90% en poids, par rapport au poids total du milieu siliconé, de composé siliconé, liquide à 25°C, ayant un paramètre de solubilité global selon l'espace de solubilité de HANSEN inférieur ou égal à 20 $(MPa)^{1/2}$, ou d'un mélange de tels composés.

**17.** Composition selon la revendication 16, dans laquelle le composé siliconé, seul ou en mélange, est choisi parmi les huiles siliconées, volatiles ou non volatiles, seules ou en mélange.

**18.** Composition selon la revendication 17, dans laquelle le composé siliconé, seul ou en mélange, est choisi parmi :

- les polydialkylsiloxanes non volatils, tels que les polydiméthylsiloxanes (PDMS) non volatils, éventuellement substitués par des groupements aliphatiques et/ou aromatiques, éventuellement fluorés, et/ou comportant des groupements fonctionnels tels que des groupements hydroxyles, thiols et/ou amines; et notamment les polysiloxanes modifiés par des acides gras (notamment en C8-C20), des alcools gras (notamment en C8-C20) ou des polyoxyalkylènes (notamment polyoxyéthyléne et/ou polyoxypropylène); les polysiloxanes aminées ; les polysiloxanes à groupement hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor
- les polydiméthylsiloxanes comportant des groupements alkyle, alcoxy ou phényle, pendant ou en bout de chaîne siliconée, ces groupements pouvant avoir de 2 à 24 atomes de carbone;
- les silicones phénylées comme les phényl triméthicones, les phényl diméthicones, les phényl triméthylsiloxy diphénylsiloxanes, les diphényl diméthicones, les diphényl méthyldiphényl trisiloxanes; et également les polyméthylphénylsiloxanes, éventuellement substitués par des groupements aliphatiques et/ou aromatiques, éventuellement fluorés, et/ou comportant des groupements fonctionnels tels que des groupements hydroxyles, thiols et/ou amines;
- les huiles siliconées cycliques ou linéaires, ayant 2 à 7 atomes de silicium, et comportant éventuellement des groupes alkyle ou alkoxy ayant de 1 à 10 atomes de carbone, telles que les cyclodiméthylsiloxanes, les cyclophénylmethylsiloxanes et les diméthylsiloxanes linéaires, et notamment la dodecamethylpentasiloxane linéaire (L5), l'octaméthylcyclotétrasiloxane (D4), le décaméthylcyclopentasiloxane (D5), le dodécaméthylcyclohexasiloxane (D6), l'heptaméthylhexyltrisiloxane, l'heptaméthyloctyltrisiloxane, l'octaméthyltrisiloxane, le décaméthyltétrasiloxane,
- leurs mélanges.

**19.** Composition selon la revendication 18, dans laquelle le composé siliconé est choisi parmi, seul ou en mélange,

- les silicones phénylées et notamment les phényl triméthicones, les phényl diméthicones, et les polyméthylphénylsiloxanes;
- les huiles siliconées volatiles, cycliques ou linéaires, ayant 2 à 7 atomes de silicium, et notamment la dodecamethylpentasiloxane linéaire (L5), l'octaméthylcyclotétrasiloxane (D4), le décaméthylcyclopentasiloxane (D5), le dodécaméthylcyclohexasiloxane (D6), et leurs mélanges.

**20.** Composition selon l'une des revendications précédentes, dans laquelle la dispersion est une dispersion de particules :

- de poly(méthacryloxypropyltris(trimethylsiloxy)silane)-b-poly(acrylate de méthyle),
- de poly(méthacryloxypropyltris(trimethylsiloxy)silane)-b-poly(acrylate de méthyle-co-acide acrylique),
- de poly(méthacryloxypropyltris(trimethylsiloxy)silane-co-acide acrylique)-b-poly(acrylate de méthyle),
- de poly(acryloxypropyltris(trimethylsiloxy)silane)-b-poly(acrylate de méthyle),
- de poly(acryloxypropyltris(trimethylsiloxy)silane)-b-poly(acrylate de méthyle-co-acide acrylique),
- de poly(acryloxypropyltris(trimethylsiloxy)silane-co-acide acrylique)-b-poly(acrylate de méthyle),
- de poly(méthacryloxypropylbis(trimethylsiloxy)méthylsilane)-b-poly(acrylate de méthyle),
- de poly(méthacryloxypropylbis(trimethylsiloxy)méthylsilane)-b-poly(acrylate de méthyle-co-acide acrylique),
- de poly(méthacryloxypropylbis(trimethylsiloxy)méthylsilane-co-acide acrylique)-b-poly(acrylate de méthyle),
- de poly(acryloxypropylbis(trimethylsiloxy)méthylsilane)-b-poly(acrylate de méthyle),
- de poly(acryloxypropylbis(trimethylsiloxy)méthylsilane)-b-poly(acrylate de méthyle-co-acide acrylique),
- de poly(acryloxypropylbis(trimethylsiloxy)méthylsilane-co-acide acrylique)-b-poly(acrylate de méthyle),
- de poly(polydimethylsiloxane méthacryloyloxy)-b-poly(acrylate de méthyle),
- de poly(polydimethylsiloxané méthacryloyloxy)-b-poly(acrylate de méthyle-co-acide acrylique),
- de poly(polydimethylsiloxane méthacryloyloxy-co-acide acrylique)-b-poly(acrylate de méthyle),
- de poly(polydimethylsiloxane méthacryloyloxy-co-méthacryloxypropyltris-(trimethylsiloxy)silane)-b-poly (acrylate de méthyle),
- de poly(polydimethylsiloxane méthacryloyloxy-co-méthacryloxypropyltris-(trimethylsiloxy)silane)-b-poly (acrylate de méthyle-co-acide acrylique),
- de poly(polydimethylsiloxane méthacryloyloxy-co-méthacryloxypropyltris-(trimethylsiloxy)silane-co-acide acrylique)-b-poly(acrylate de méthyle),
- de poly(polydimethylsiloxane méthacryloyloxy-co-acryloxypropyltris-(trimethylsiloxy)silane)-b-poly(acrylate de méthyle),
- de poly(polydimethylsiloxane méthacryloyloxy-co-acryloxypropyltris-(trimethylsiloxy)silane)-b-poly(acrylate de méthyle-co-acide acrylique),
- de poly(polydimethylsiloxane méthacryloyloxy-co-acryloxypropyltris-(trimethylsiloxy)silane-co-acide acrylique)-b-poly(acrylate de méthyle),
- de poly(méthacryloxypropylbis(trimethylsiloxy)méthylsilane-co-méthacryloxypropyltris(trimethylsiloxy)silane)-b-poly(acrylate de méthyle),
- de poly(méthacryloxypropylbis(trimethylsiloxy)méthylsilane-co-méthacryloxypropyltris(trimethylsiloxy)silane)-b-poly(acrylate de méthyle-co-acide acrylique),
- de poly(méthacryloxypropylbis(trimethylsiloxy)méthylsilane-co-méthacryloxypropyltris(trimethylsiloxy)silane-co-acide acrylique)-b-poly(acrylate de méthyle),
- de poly(méthacryloxypropylbis(trimethylsiloxy)méthylsilane-co-acryloxypropyltris(trimethylsiloxy)silane)-b-poly(acrylate de méthyle),
- de poly(méthacryloxypropylbis(trimethylsiloxy)méthylsilane-co-acryloxypropyltris(trimethylsiloxy)silane)-b-poly(acrylate de méthyle-co-acide acrylique),
- de poly(méthacryloxypropylbis(trimethylsiloxy)méthylsilane-co-acryloxypropyltris(trimethylsiloxy)silane-co-acide acrylique)-b-poly(acrylate de méthyle),
- de poly(méthacryloxy-propyltris(trimethylsiloxy)silane-co-acryloxypropyltris(trimethylsiloxy)silane)-b-poly (acrylate de méthyle),
- de poly(méthacryloxy-propyltris(trimethylsiloxy)silane-co-acryloxypropyltris(trimethylsiloxy)silane)-b-poly (acrylate de méthyle-co-acide acrylique),
- de poly(méthacryloxy-propyltris(trimethylsiloxy)silane-co-acryloxypropyltris(trimethylsiloxy)silane-co-acide acrylique)-b-poly(acrylate de méthyle),
- de poly(polydimethylsiloxane méthacryloyloxy-co-méthacryloxypropylbis(trimethylsiloxy)méthylsilane)-b-poly (acrylate de méthyle)
- de poly(polydimethylsiloxane méthacryloyloxy-co-méthacryloxypropylbis(trimethylsiloxy)méthylsilane)-b-poly (acrylate de méthyle-co-acide acrylique)
- de poly(polydimethylsiloxane méthacryloyloxy-co-méthacryloxypropylbis(trimethylsiloxy)méthylsilane-co-acide acrylique)-b-poly(acrylate de méthyle);
- de poly(polydimethylsiloxane méthacryloyloxy-co-acryloxypropylbis(trimethylsiloxy)méthylsilane)-b-poly (acrylate de méthyle)
- de poly(polydimethylsiloxane méthacryloyloxy-co-acryloxypropylbis(trimethylsiloxy)méthylsilane)-b-poly (acrylate de méthyle-co-acide acrylique)
- de poly(polydimethylsiloxane méthacryloyloxy-co-acryloxypropylbis(trimethylsiloxy)méthylsilane-co-acide acrylique)-b-poly(acrylate de méthyle);
- de poly(méthacryloxypropyltris(trimethylsiloxy)silane)-b-poly(acrylate de méthyle)-b- poly(méthacryloxypropyltris(trimethylsiloxy)silane,
- de poly(méthacryloxypropyltris(trimethylsiloxy)silane-co-acide acrylique)-b-poly(acrylate de méthyle)-b- poly(méthacryloxypropyltris(trimethylsiloxy)silane-co-acide acrylique),
- de poly(méthacryloxypropyltris(trimethylsiloxy)silane)-b-poly(acrylate de méthyle-co-acide acrylique)-b- poly(méthacryloxypropyltris(trimethylsiloxy)silane),
- de poly(acryloxypropyltris(trimethylsiloxy)silane)-b-poly(acrylate de méthyle)-b-poly(acryloxypropyltris(trimethylsiloxy)silane),
- de poly(acryloxypropyltris(trimethylsiloxy)silane-co-acide acrylique)-b-poly(acrylate de méthyle)-b- poly(acryloxypropyltris(trimethylsiloxy)silane-co-acide acrylique),
- de poly(acryloxypropyltris(trimethylsiloxy)silane)-b-poly(acrylate de méthyle-co-acide acrylique)-b- poly(acryloxypropyltris(trimethylsiloxy)silane),
- de poly(méthacryloxypropylbis(trimethylsiloxy)méthylsilane)-b-poly(acrylate de méthyle)-b- poly(méthacryloxypropylbis(trimethylsiloxy)méthylsilane);
- de poly(méthacryloxypropylbis(trimethylsiloxy)méthylsilane-co-acide acrylique)-b-poly(acrylate de méthyle)-b-poly(méthacryloxypropylbis(trimethylsiloxy)-méthylsilane-co-acide acrylique);
- de poly(méthacryloxypropylbis(trimethylsiloxy)méthylsilane)-b-poly(acrylate de méthyle-co-acide acrylique)-b-poly(méthacryloxypropylbis(trimethylsiloxy) méthylsilane)
- de poly(acryloxypropylbis(trimethylsiloxy)méthylsilane)-b-poly(acrylate de méthyle)-b- poly(acryloxypropylbis(trimethylsiloxy)méthylsilane)
- de poly(acryloxypropylbis(trimethylsiloxy)méthylsilane-co-acide acrylique)-b-poly(acrylate de méthyle)-b- poly(acryloxypropylbis(trimethylsiloxy)méthylsilaneco-acide acrylique)
- de poly(acryloxypropylbis(trimethylsiloxy)méthylsilane)-b-poly(acrylate de méthyle-co-acide acrylique)-b- poly(acryloxypropylbis(trimethylsiloxy)méthylsilane)
- de poly(polydimethylsiloxane méthacryloyloxy)-b-poly(acrylate de méthyle)-b-poly(polydimethylsiloxane méthacryloyloxy)
- de poly(polydimethylsiloxane méthacryloyloxy-co-acide acrylique)-b-poly(acrylate de méthyle)-b- poly(polydimethylsiloxane méthacryloyloxy-co-acide acrylique)
- de poly(polydimethylsiloxane méthacryloyloxy)-b-poly(acrylate de méthyle-co-acide acrylique)-b- poly(polydimethylsiloxane méthacryloyloxy)
- de poly(polydimethylsiloxane méthacryloyloxy-co-méthacryloxypropyltris-(trimethylsiloxy)silane)-b-poly(acrylate de méthyle)-b-poly(polydimethylsiloxane méthacryloyloxy-co-méthacryloxypropyltris-(trimethylsiloxy)sitane)
- de poly(polydimethylsiloxane méthacryloyloxy-co-méthacryloxypropyltris-(trimethylsiloxy)silane-co-acide acrylique)-b-poly(acrylate de méthyle)-b-poly(polydimethylsiloxane méthacryloyloxy-co-méthacryloxypropyltris-(trimethylsiloxy)silane-co-acide acrylique)
- de poly(polydimethylsiloxane méthacryloyloxy-co-méthacryloxypropyltris-(trimethylsiloxy)silane)-b-poly(acrylate de méthyle-co-acide acrylique)-b-poly(polydimethylsiloxane méthacryloyloxy-co-méthacryloxypropyltris-(trimethylsiloxy)silane)
- de poly(polydimethylsiloxane méthacryloyloxy-co-acryloxypropyltris-(trimethylsiloxy)silane)-b-poly(acrylate de méthyle)-b-poly(polydimethylsiloxane méthacryloyloxy-co-acryloxypropyltris-(trimethylsiloxy)silane)
- de poly(polydimethylsiloxane méthacryloyloxy-co-acryloxypropyltris-(trimethylsiloxy)silane-co-acide acrylique)-b-poly(acrylate de méthyle)-b-poly(polydimethylsiloxane méthacryloyloxy-co-acryloxypropyltris-(trimethylsiloxy)silane-co-acide acrylique),
- de poly(polydimethylsiloxane méthacryloyloxy-co-acryloxypropyltris-(trimethylsiloxy)silane)-b-poly(acrylate de méthyle-co-acide acrylique)-b- poly(polydimethylsiloxane méthacryloyloxy-co-acryloxypropyltris-(trimethylsiloxy)silane),
- de poly(méthacryloxy-propyltris(trimethylsiloxy)silane-co-acryloxypropyltris-(trimethylsiloxy)silane)-b-poly(acrylate de méthyle)-b-poly(méthacryloxypropyltris(trimethylsiloxy)silane-co-acryloxy-propyltris(trimethylsiloxy)silane)
- de poly(méthacryloxy-propyltris(trimethylsiloxy)silane-co-acryloxypropyltris-(trimethylsiloxy)silane-co-acide acrylique)-b-poly(acrylate de méthyle)-b-poly(méthacryloxy-propyltris(trimethylsiloxy)silane-co-acryloxypropyltris(trimethylsiloxy)silane-co-acide acrylique)
- de poly(méthacryloxypropyltris(trimethylsiloxy)silane-co-acryloxypropyltris(trimethylsiloxy)silane)-b-poly(acrylate de méthyle-co-acide acrylique)-b-poly(méthacryloxypropyltris(trimethylsiloxy)silane-co-acryloxypropyltris(trimethylsiloxy)silane);

dans un milieu siliconé, plus particulièrement dans une silicone phénylée et/ou une huile de silicone volatile, et notamment dans une huile de silicone volatile cyclique telle que la D5.

**21.** Composition selon l'une des revendications précédentes, dans laquelle la dispersion présente un taux de matière sèche compris entre 5 et 80% en poids, notamment 8 à 70% en poids, voire 10 à 60%, ou encore 15 à 50% en poids, et mieux 18 à 25% en poids.

**22.** Composition selon l'une des revendications précédentes, dans laquelle la dispersion est présente en une quantité de 0,1 à 90% en poids, de préférence 0,5 à 80% en poids, notamment 1 à 75% en poids, voire 5-70% en poids, de dispersion par rapport au poids total de la composition.

**23.** Composition selon l'une des revendications précédentes, comprenant en outre, au moins un constituant choisi parmi les phases grasses, les phases hydrophiles, les matières colorantes, les polymères, les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents antipelliculaires, les agents propulseurs, les céramides, ou leurs mélanges.

**24.** Composition selon l'une des revendications précédentes, se présentant sous forme d'une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux); d'une composition de protection ou de soin de la peau du visage, du cou, des mains ou du corps, notamment une composition anti-rides, anti-fatigue permettant de donner un coup d'éclat à la peau, une composition hydratante ou traitante; une composition anti-solaire ou de bronzage artificiel; d'un produit capillaire, notamment pour le maintien de la coiffure ou la mise en forme des cheveux.

**25.** Dispersion de particules de polymères dans un milieu siliconé liquide, ledit polymère étant un copolymère séquencé comprenant au moins une première séquence soluble dans ledit milieu siliconé et au moins une deuxième séquence insoluble dans ledit milieu siliconé, la dispersion étant telle que définie à l'une quelconque des revendications 1 à 21.

**26.** Procédé cosmétique de maquillage, de nettoyage, de protection solaire, de mise en forme, de coloration, ou de soin des matières kératiniques, notamment de la peau du corps ou du visage, des ongles, des cheveux et/ou des cils, comprenant l'application sur lesdites matières d'une composition cosmétique selon l'une des revendications 1 à 24.

Office européen des brevets

# RAPPORT DE RECHERCHE EUROPEENNE

Numéro de la demande
EP 06 29 0132

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X<br><br><br><br>D | WO 2004/055077 A (L'OREAL; ARNAUD, PASCAL; LION, BERTRAND)<br>1 juillet 2004 (2004-07-01)<br>* revendications 1-8,39-44,52-83 *<br>& EP 1 428 843 A 16 juin 2004 (2004-06-16)<br>----- | 1-26 | INV.<br>A61K8/89<br>A61K8/90 |
| X<br><br><br>D | WO 2004/055081 A (L'OREAL; DE LA POTERIE, VALERIE) 1 juillet 2004 (2004-07-01)<br>* revendications 1-24,37-65,71,75 *<br>& EP 1 428 843 A 16 juin 2004 (2004-06-16)<br>----- | 1-26 | |
| X | US 5 804 173 A (HUTCHINS ET AL)<br>8 septembre 1998 (1998-09-08)<br>* colonne 26; exemple 1 *<br>* colonne 28, ligne 43 - ligne 44 *<br>* exemples 15-18,21 *<br>----- | 1,25 | |
| X | EP 0 963 751 A (DOW CORNING TORAY SILICONE COMPANY, LTD)<br>15 décembre 1999 (1999-12-15)<br>* page 2, ligne 31 - page 10, ligne 55 *<br>* page 11, ligne 9 - ligne 10 *<br>* revendications *<br>----- | 1,7-11,<br>13-19,<br>23-26 | DOMAINES TECHNIQUES RECHERCHES (IPC)<br>A61K |
| A | DATABASE WPI<br>Section Ch, Week 200456<br>Derwent Publications Ltd., London, GB;<br>Class A14, AN 2004-573565<br>XP002386588<br>& JP 2004 203917 A (MITSUBISHI CHEM CORP)<br>22 juillet 2004 (2004-07-22)<br>* abrégé *<br>----- | 1-26 | |

Le présent rapport a été établi pour toutes les revendications

| Lieu de la recherche | Date d'achèvement de la recherche | Examinateur |
|---|---|---|
| Berlin | 26 juin 2006 | Pelli Wablat, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

7

**ANNEXE AU RAPPORT DE RECHERCHE EUROPEENNE
RELATIF A LA DEMANDE DE BREVET EUROPEEN NO.** EP 06 29 0132

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

26-06-2006

| Document brevet cité au rapport de recherche | Date de publication | Membre(s) de la famille de brevet(s) | Date de publication |
|---|---|---|---|
| WO 2004055077 A | 01-07-2004 | AU 2003300595 A1 | 09-07-2004 |
| | | AU 2003300596 A1 | 09-07-2004 |
| | | AU 2003300597 A1 | 09-07-2004 |
| | | AU 2003300598 A1 | 09-07-2004 |
| | | AU 2003300599 A1 | 09-07-2004 |
| | | AU 2003300601 A1 | 09-07-2004 |
| | | AU 2003300602 A1 | 09-07-2004 |
| | | AU 2003301500 A1 | 09-07-2004 |
| | | EP 1572770 A2 | 14-09-2005 |
| | | EP 1585777 A2 | 19-10-2005 |
| | | EP 1583784 A2 | 12-10-2005 |
| | | EP 1572137 A2 | 14-09-2005 |
| | | WO 2004055073 A2 | 01-07-2004 |
| | | WO 2004055078 A1 | 01-07-2004 |
| | | WO 2004055079 A2 | 01-07-2004 |
| | | WO 2004055074 A2 | 01-07-2004 |
| | | WO 2004055080 A2 | 01-07-2004 |
| | | WO 2004055081 A2 | 01-07-2004 |
| | | WO 2004055082 A2 | 01-07-2004 |
| | | JP 2006509809 T | 23-03-2006 |
| | | JP 2006509810 T | 23-03-2006 |
| | | JP 2006509811 T | 23-03-2006 |
| | | JP 2006509812 T | 23-03-2006 |
| EP 1428843 A | 16-06-2004 | FR 2848560 A1 | 18-06-2004 |
| | | JP 2004190035 A | 08-07-2004 |
| WO 2004055081 A | 01-07-2004 | AU 2003300595 A1 | 09-07-2004 |
| | | AU 2003300596 A1 | 09-07-2004 |
| | | AU 2003300597 A1 | 09-07-2004 |
| | | AU 2003300598 A1 | 09-07-2004 |
| | | AU 2003300599 A1 | 09-07-2004 |
| | | AU 2003300601 A1 | 09-07-2004 |
| | | AU 2003300602 A1 | 09-07-2004 |
| | | AU 2003301500 A1 | 09-07-2004 |
| | | EP 1572770 A2 | 14-09-2005 |
| | | EP 1585777 A2 | 19-10-2005 |
| | | EP 1583784 A2 | 12-10-2005 |
| | | EP 1572137 A2 | 14-09-2005 |
| | | WO 2004055077 A2 | 01-07-2004 |
| | | WO 2004055073 A2 | 01-07-2004 |
| | | WO 2004055078 A1 | 01-07-2004 |
| | | WO 2004055079 A2 | 01-07-2004 |
| | | WO 2004055074 A2 | 01-07-2004 |
| | | WO 2004055080 A2 | 01-07-2004 |
| | | WO 2004055082 A2 | 01-07-2004 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE RELATIF A LA DEMANDE DE BREVET EUROPEEN NO.

EP 06 29 0132

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

26-06-2006

| Document brevet cité au rapport de recherche | Date de publication | Membre(s) de la famille de brevet(s) | Date de publication |
|---|---|---|---|
| WO 2004055081 A | | JP 2006509809 T | 23-03-2006 |
| | | JP 2006509810 T | 23-03-2006 |
| | | JP 2006509811 T | 23-03-2006 |
| | | JP 2006509812 T | 23-03-2006 |
| EP 1428843 A | 16-06-2004 | FR 2848560 A1 | 18-06-2004 |
| | | JP 2004190035 A | 08-07-2004 |
| US 5804173 A | 08-09-1998 | AUCUN | |
| EP 0963751 A | 15-12-1999 | CA 2273547 A1 | 12-12-1999 |
| | | DE 69919780 D1 | 07-10-2004 |
| | | DE 69919780 T2 | 15-09-2005 |
| | | US 6280748 B1 | 28-08-2001 |
| JP 2004203917 A | 22-07-2004 | AUCUN | |

EPO FORM P0460

Pour tout renseignement concernant cette annexe : voir Journal Officiel de l'Office européen des brevets, No.12/82